(19) Europäisches Patentamt
European Patent Office
Office européen des brevets

(11) **EP 2 504 301 B1**

(12) **EUROPÄISCHE PATENTSCHRIFT**

(45) Veröffentlichungstag und Bekanntmachung des Hinweises auf die Patenterteilung:
**02.07.2014 Patentblatt 2014/27**

(51) Int Cl.:
***C07C 29/80*** *(2006.01)*     ***C07C 31/20*** *(2006.01)*
***C07C 27/28*** *(2006.01)*

(21) Anmeldenummer: **10781500.3**

(22) Anmeldetag: **22.11.2010**

(86) Internationale Anmeldenummer:
**PCT/EP2010/067881**

(87) Internationale Veröffentlichungsnummer:
**WO 2011/064155 (03.06.2011 Gazette 2011/22)**

(54) **VERFAHREN ZUR HERSTELLUNG VON 1,6-HEXANDIOL**

METHOD FOR PRODUCING 1,6-HEXANEDIOL

PROCÉDÉ DE PRÉPARATION DE 1,6-HEXANEDIOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.11.2009 DE 102009047196**

(43) Veröffentlichungstag der Anmeldung:
**03.10.2012 Patentblatt 2012/40**

(73) Patentinhaber: **BASF SE**
**67056 Ludwigshafen (DE)**

(72) Erfinder: **PINKOS, Rolf**
**67098 Bad Dürkheim (DE)**

(56) Entgegenhaltungen:
**WO-A1-97/31882     US-B1- 6 426 438**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft ein Verfahren zur Herstellung von 1,6-Hexandiol, wobei ein Hexandiol mit einem Stickstoffgewichtsanteil von weniger als 5 ppm erhalten wird.

WO 97/31882 A1 und US 6,426,438 beschreiben Verfahren zur Gewinnung von 1,6-Hexandiol durch Hydrierung von Dicarbonsäureestern und anschließende destillative Reinigung des 1,6-Hexandiols.

Es besteht ein großer Bedarf an 1,6-Hexandiol, das für die Herstellung von Polyurethanen keine katalytischen Mengen an Aminen aufweist, da diese katalytischen Mengen von Aminen zu erheblichen Nebenprodukten führen, die die Umsetzung zum Polyurethan behindern.

DE 10112117 A1 beschreibt ein Verfahren zur Entfernung von stickstoffhaltigen Verbindungen unter Verwendung von sauren und/oder basischen Ionenaustauschern. Der Stickstoffgewichtsanteil wird hierbei mittels eines CPR-Werts (controlled polymerization rate) ermittelt. Dieses Reinigungsverfahren hat den Nachteil, dass der Einsatz von sauren und/oder basischen Ionenaustauschern zu erhöhten Kosten führt, da die Ionenaustauscher an sich Kosten bedeuten, als auch ihr Einsatz ein erhöhten Einsatz von Lösungsmitteln bedeuten, da die Ionenaustauscher nur endlich einsetzbar sind und immer wieder regeneriert werden müssen. Dazu ist, um Produktverluste zu vermeiden, ein Spülen des Ionentauschers notwendig, was zusätzlichen Einsatz von Lösungsmitteln bzw. Regeneriermedien erfordert. Da 1,6-Hexandiol unter Normalbedingungen fest ist, muss der Zulauf zum Ionenaustauscher zusätzlich beheizt werden, damit eine Umsetzung am Ionenaustauscher überhaupt möglich ist. Somit weist das Verfahren zur Reinigung von Polyalkoholen in D2 101112114 A1 erhebliche Nachteile auf. Des Weiteren wird in DE 101112114 A1 die Entfernung von stickstoffhaltigen Verbindungen aus 1,6-Hexandiol nicht beschrieben.

Bei der Herstellung von 1,6-Hexandiol wird von den entsprechenden Cyclo-$C_6$-Alkanen, Alkoholen, Ketonen und/oder Mischungen dieser Verbindungen ausgegangen, die entweder in Gegenwart von Salpetersäure oxidiert werden und/oder einer Oxidation mit anschließender Wasserextraktion des organischen Stoffstromes unterzogen werden.

Bei 1,6-Hexandiol werden so Adipinsäure enthaltende Ströme produziert, die z. B. ausgehend von Cyclohexanol und/oder Cyclohexanon durch Oxidation mit Salpetersäure gewonnen werden. Unter Adipinsäure enthaltende Ströme versteht man solche, die Adipinsäure selbst aber auch Adipinsäure in Form ihrer Ester enthalten können. Bei der Oxidation kann sowohl die durch die Oxidation gewonnene Adipinsäure, als auch das nach weitgehender Abtrennung der Adipinsäure übrigbleibende Gemisch, das Adipinsäure, Glutarsäure und Bernsteinsäure enthält, eingesetzt werden.

Ferner sind prinzipiell auch andere Quellen für Adipinsäure oder adipinsäurehaltige Stoffströme zu nennen, die auch mit den vorgenannten Stoffströmen gemischt werden können, wie z.B. solche, die man durch Oxidation von Cyclohexan zu Cyclohexanol/Cyclohexanon-Gemischen und anschließende Wasserextraktion des organischen Stoffstromes erhält.

Üblicherweise enthalten die vorgenannten Ströme Verunreinigungen, die im Falle der Oxidation von Cyclohexanol/Cyclohexanon durch die Oxidation mit Salpetersäure entstehen und Stickstoff enthalten. Auch in den Wasserextrakten nach der Oxidation von Cyclohexan mit Luft finden sich Stickstoff-Komponenten als unerwünschte Nebenkomponenten.

Diese Stickstoffverbindungen, die beispielsweise als Nitrogruppe, Amide oder als Ammoniumion vorliegen können, sind in der Lage, während der Hydrierung von adipinsäurehaltigen Strömen, die auch verestert sein können, Amine zu bilden. Beispielsweise können Nitroverbindungen direkt zu Aminen und/oder Amiden hydriert werden. Ammoniumionen können während der Hydrierung entstandene Alkohole aminieren.

Amine sind basische Komponenten und sind als solche in 1,6-Hexandiol unerwünscht, da sie bei den Anwendungen unerwünschte Eigenschaften haben. So können diese Amine beispielsweise bei der Herstellung von Polyurethanen katalytisch wirken, so dass die Prozesskontrolle zur Herstellung eines Produktes mit genau definierten Eigenschaften schwierig, wenn nicht gar unmöglich ist. Es kann vorkommen, dass ganze Produktionschargen entsorgt werden müssen. Prinzipiell gilt das auch bei der Herstellung von Polyestern oder Polyesteralkohole, die dann wiederum weiter mit Isocyanaten zu Urethanen umgesetzt werden.

Eine Möglichkeit festzustellen, ob in 1,6-Hexandiol unerwünschte N-haltige Verbindungen in Form von basisch wirkenden Aminen vorhanden sind, ist die Bestimmung des sog. CPR-Werts (controlled polymerization rate). Der Gehalt von basisch wirkendem Amin ist folgendermaßen mit dem CPR-Wert gekoppelt und kann, wie am Beispiel von 1,6-Hexandiol erklärt, bestimmt werden:

30 g 1,6-Hexandiol werden in 100 ml einer Lösung von Kaliumhydroxid in Methanol (0,001 mol/l) gelöst und 15 min gerührt. Diese Lösung wird beispielsweise mit einem Titroprozessor 682 TM der Firma Metrohm, Herison, Schweiz, potentiometrisch mit 0,01 N Salzsäure bis zum Endpunkt titriert. Der Titroprozessor 682 ist mit zwei pH-Elektroden, einer Glaselektrode (3 M KCI, Metrohm 6.0133.100) und einer Ag/AgCl/LiCl-Elektorde (Alkohol, Metrohm 6.0726.100) ausgestattet. Mit einer Vergleichslösung bestehend aus 100 ml einer Lösung von Kaliumhydroxid in Methanol (0,001 mol/l) wird entsprechend verfahren, um den Blindwert zu bestimmen.

Der CPR-Wert wird aus beiden Ergebnissen der potentiometrischen Titration wie folgt bestimmt:

$$CPR = 10 \times (V1 - V2), \text{ wobei}$$

wobei

V1 dem Verbrauch an 0,01 N Salzsäure in der Polyalkohol-Probe,
V2 dem Verbrauch im Vergleich (Blindwert) und
10 dem Berechnungsfaktor in Übereinstimmung mit JIS (Japan Industrial Standard) K 1557-1970 entspricht.

Beispielsweise befinden sich bei einem CPR-Wert von 10, d.h. einem netto Salzsäureverbrauch von 1 g 0,01 molarer HCL ca. 5 ppm N im 1,6 Hexandiol. Ein solcher CPR-Wert von 10, d.h. ein N-Gehalt von 5 ppm stellt bereits ein unerwünscht hohes Niveau dar und kann in nachfolgenden Polyurethanreaktionen schon erhebliche Nebenreaktionen verursachen.

Die Aufgabe der vorliegenden Erfindung ist es daher, ein Verfahren bereit zu stellen, das es ermöglicht, 1,6-Hexandiol herzustellen, das einen CPR-Wert von kleiner 10 aufweist, ohne erhöhten Mehraufwand und Kosten mit zusätzlichen Lösungsmitteln und/oder sauren und/oder basischen Ionenaustauschern aufbringen zu müssen. Diese Aufgabe wird gelöst durch ein Verfahren zur Reinigung von 1,6-Hexandiol umfassend folgende Schritte

I) Bereitstellung einer Mischung, die 1,6-Hexandiol enthält
II) Destillation dieser Mischung aus Schritt I
III) Auffangen eines 1,6-Hexandiols mit einem Stickstoffanteil von weniger als 5 ppm,
IV) Ausschleusen von zumindest 50 % der stickstoffhaltige Komponenten mit dem hochsiedenden Sumpfstrom,

wobei vor und/oder während der Destillation in Schritt II mehr als 500 ppm an Carbonsäuren und/oder Ester enthalten sind, die einen höheren Siedepunkt als 1,6-Hexandiol aufweisen und bei Temperaturen von $\geq$ 100°C wenigstens 5 min lang in Kontakt mit dem 1,6-Hexandiol stehen.

Bei dem erfindungsgemäßen Verfahren ist es notwendig, dass die zu destillierende Mischung aus Schritt I des erfindungsgemäßen Verfahrens neben 1,6-Hexandiol auch Carbonsäuren und/oder Ester enthält, die einen höheren Siedepunkt als 1,6-Hexandiol selber aufweisen. Dies kann dadurch erreicht werden, dass die in Schritt I) eingesetzte Mischung neben dem 1,6-Hexandiol bereits Carbonsäuren und/oder Ester enthält, die mit 1,6-Hexandiol höhersiedende Ester bildet oder aber vor und/oder während der Destillation in Schritt II, der Mischung aus Schritt I entweder Carbonsäuren und/oder Ester, die einen höheren Siedpunkt als 1,6-Hexandiol aufweisen (Hochsieder), zugesetzt werden oder aber Carbonsäuren und/oder Estern zugegeben werden, die mit einem Teil des 1,6-Hexandiols zu Estern umgesetzt werden, die nach der Umsetzung einen höheren Siedepunkt als 1,6-Hexandiol aufweisen. Es können auch Mischungen aus Hochsiedern und Carbonsäuren und/oder Estern, die mit 1,6-Hexandiol höher als 1,6-Hexandiol selber siedende Ester bilden, eingesetzt werden. Wenn vor und/oder während der eigentlichen Destillation nach Schritt II solche Hochsieder oder Carbonsäuren und/oder Ester hinzugegeben werden, die mit dem 1,6-Hexandiol selber solche höhersiedenden Ester bilden, dann muss die Destillation so geführt werden, dass diese Hochsieder und/oder höhersiedenden Ester mit dem 1,6-Hexandiol in der Mischung aus Schritt I für eine bestimmte Zeit in Kontakt stehen, bevor das 1,6-Hexandiol abdestilliert wird. Diese Kontaktzeit muss während der Destillation bei einem Temperaturbereich von $\geq$ 100°C wenigstens 5 min betragen. Bevorzugt ist eine Kontaktzeit von $\geq$ 10 Minuten, besonders bevorzugt ist eine Kontaktzeit von $\geq$ 15 Minuten. Unter Kontaktzeit versteht man die Zeit, in der das 1,6-Hexandiol mit dem Hochsieder und/oder den höhersiedenden Estern innerhalb der Kolonne in flüssigem oder gasförmigen Zustand in Kontakt steht. Der Kontaktraum in dem das 1,6-Hexandiol mit dem Hochsieder und/oder den höhersiedenden Estern in Kontakt stehen muss, ist die gesamte Kolonne sowie die dazu gehörigen Rohrleitungen und gegebenenfalls der Verdampfer. Der Kontaktraum umfasst somit die Packung innerhalb der Kolonne, die Sammler und Verteiler, sowie die hierzu gehörigen Rohrleitungen, den Kolonnensumpf als auch einen gegebenenfalls angeschlossenen Verdampfer und die Rohrleitung zu diesem.

Die Temperatur während der Kontaktzeit sollte $\geq$ 100°C, bevorzugt mindestens 120°C, besonders bevorzugt mindestens 140°C sein.

Die Carbonsäuren und/oder Estern die gegebenenfalls der Mischung aus Schritt I vor der Destillation nach Schritt II zugegeben werden, sind dabei ausgewählt aus der Gruppe von Adipinsäure, Adipinsäureestern, 6-Hydroxycapronsäure, 6-Hydroxycapronsäureestern. Besonders bevorzugt sind die Ester, die ausgewählt sind aus der Gruppe von Adipinsäuredimethylester, 6-Hydroxycapronsäuremethylester, Adipinsäure-1,6-hexandiolmethlyester, Adipinsäure-di-1,6-hexandiolester, 6-Hydroxycapronsäure-1,6-hexandiolester und Mischungen dieser Ester.

Die Menge der Carbonsäuren und/oder Estern, die mit dem 1,6-Hexandiol zu den höhersiedenden Estern umgesetzt werden als auch die Menge an zugesetzten Hochsiedern sowie die Menge an Mischungen aus zugesetzten Hochsiedern und Carbonsäuren und/oder Estern, die mit dem 1,6-Hexandiol zu den höhersiedenden Estern umgesetzt werden, liegen im Bereich $\geq$500 ppm, bevorzugt liegt die Menge im Bereich von $\geq$1000 ppm, besonders bevorzugt $\geq$ 1500 ppm, bezogen auf die Menge an zu destillierendem 1,6-Hexandiol.

Die Drücke bei der Destillation liegen bevorzugt zwischen 5 und 3000 mbar absolut. Bevor es zur eigentlichen Destillation nach Schritt II dies erfindungsgemäßen Verfahrens kommt, können gegebenenfalls andere Verbindungen vorher abdestilliert werden. Dies sind vor allem solche Verbindungen, die einen um mindestens 50°C niedrigeren Siedepunkt haben als das 1,6-Hexandiol selbst und als Leichtsieder bezeichnet werden. Bevorzugt sind die Leichtsieder ausgewählt aus der Gruppe von Methanol, Wasser, Dimethylether, 1-Hexanol und 1-Methoxy-6-hydroxyhexan. Die Abtrennung der Leichtsieder kann in einer gesonderten Kolonne erfolgen, die der Destillation nach Schritt II vorgelagert ist. Innerhalb dieser Kolonne zur Abtrennung der Leichtsieder liegt der Druck, wenn beispielsweise Methanol und/oder Wasser abgetrennt werden soll, zwischen 200 und 3000 mbar absolut, wenn 1,6-Hexandiol von Hochsiedern und/oder den höhersiedenden Estern abgetrennt werden soll, so liegt der Druck während dieser Destillation zwischen 5, bevorzugt 10 und 500 mbar absolut, bevorzugt zwischen 20 und 300 mbar absolut, besonders bevorzugt zwischen 30 und 200 mbar absolut.

Die Destillationen können als batch-Prozess oder kontinuierlich erfolgen, bevorzugt ist allerdings der kontinuierliche Betrieb, vor allem dann, wenn das 1,6-Hexandiol in technischen Mengen produziert werden soll.

Zur Herstellung von 1,6-Hexandiol mit einem Stickstoffgewichtsanteil von kleiner 5 ppm wird von Adipinsäure ausgegangen, die entweder durch Oxidation von Cyclohexanol und/oder Cyclohexanon mittels Salpetersäure, durch Oxidation von Cyclohexan zu Cyclohexanol/Cyclohexanon-Gemischen und anschließende Wasserextraktion des organischen Stoffstromes oder durch Oxidation von Cyclohexan mit Luft und anschließender Wasserextraktion hergestellt wurde. Hierbei wird die in diesen Lösungen enthaltene Adipinsäure mit einem Alkohol ausgewählt aus der Gruppe von Methanol, Ethanol, Propanol, iso-Propanol, n-Butanol, iso-Butanol, Pentanole, Hexanole, 2-Ethylhexanol, 2-Propylheptanol, 1,5-Pentandiol, 1,6-Hexandiol, Tridecanol, Pentadecanol oder Mischungen aus den Alkoholen, bevorzugt Methanol, Ethanol, Propanol, n-Butanol und 1,6-Hexandiol verestert. Besonders bevorzugt sind Methanol und 1,6-Hexandiol zur Veresterung. Die anschließende Hydrierung kann in der Gasphase oder in der Flüssigphase erfolgen.

Wenn die nachfolgende Hydrierung in der Gasphase erfolgen soll, ist Methanol als Alkohol zur Veresterung bevorzugt.

Soll in der Flüssigphase hydriert werden, ist neben Methanol auch 1,6-Hexandiol bevorzugt.

Dabei wird der Alkohol mindestens äquimolar zu den Carboxylgruppen der Adipinsäure und gegebenenfalls anderen anwesenden Carboxylgruppen anderer Säuren eingesetzt. Bevorzugt ist jedoch ein molarer Alkoholüberschuss pro Carboxylgruppe von mindestens 2.

Dabei kann die Veresterung ohne zugesetzten Katalysator ablaufen, bevorzugt ist jedoch, zumindest nach einem Säureumsatz von 50 Gew.-%, einen Katalysator zu verwenden. Dies können beispielsweise Schwefelsäure oder Sulfonsäuren sein, jedoch auch saure Feststoffe wie z.B. Ionentauscher, meist auf Sulfonsäurebasis.

Das entstehende Reaktionswasser wird vorzugsweise während der Veresterung, z.B. destillativ, abgetrennt. Dabei wird auch Alkohol mitgeführt. Deshalb ist es bevorzugt, das Alkohol-Wasser-Gemisch separat aufzudestillieren und den Alkohol zurückzuführen.

Je nach Veresterungstechnologie kann der Adipinsäuredialkylester bereits für die Hydrierung einsatzfähig anfallen, es kann aber auch sein, dass noch Alkohol und Wasser abgetrennt werden müssen bzw. der Adipinsäuredialkylester destillativ gereinigt werden muss, um von nicht vollständig umgesetzter Säure abzutrennen, die entweder entsorgt wird, oder bevorzugt, gegebenenfalls unter Ausschleusung eines geringen Prozentsatzes, zur Vermeidung von Aufpegelung, von unerwünschten Komponenten, in die Veresterung rückgeführt wird. Der gegebenenfalls gereinigte Adipinsäuredialkylester wird anschließend hydriert.

Dies kann in der Flüssigphase oder Gasphase bevorzugt an Cu-haltigen Katalysatoren geschehen. Bei der Flüssigphasenhydrierung werden bevorzugt Drücke von 100 - 330 bar absolut angewendet, bevorzugt sind 150 - 270 bar Überdruck, in der Gasphase sind 5 bis 100 bar Überdruck sinnvoll, besonders bevorzugt sind 20 bis 70 bar.

Vorteilhaft ist es, wenn die Hydrierung im Hydrieraustrag noch Carboxylgruppen, bevorzugt Ester enthält. Dies können z.B. Adipinsäuredimethyester, 6-Hydroxycapronsäuremethylester, Adipinsäure-1,6-hexandiol-methylester, Adipinsäuredi-1,6-hexandiolester und/oder 6-Hydroxcapronsäure-1,6-hexandiolester sein. Sollten ausschließlich oder nahezu ausschließlich Adipinsäuredimethyester und/oder 6-Hydroxycapronsäuremethylester als Carboxylgruppen-haltige Verbindungen im Hydrieraustrag sein, so ist in der oder den nachfolgenden Destillationsstufen, oder in einer separaten Stufe dafür zu sorgen, dass diese Ester zumindest nicht vollständig von 1,6-Hexandiol abdestilliert werden. Gemäß dem erfindungsgemäßen Verfahren werden diese Ester mit dem 1,6-Hexandiol bei Temperaturen von ≥ 100°C zu entsprechenden höher als 1,6-Hexandiol selbst siedenden Estern umgesetzt. Diese entsprechenden höhersiedenden Ester sind ausgewählt aus der Gruppe von Adipinsäure-1,6-hexandiol-methylester, Adipinsäuredi-1,6-hexandiolester und/oder 6-Hydroxcapronsäure-1,6-hexandiolester. Der Gehalt dieser hochersiedenden Ester bezogen auf den Gehalt an 1,6-Hexandiol beträgt zumindest 500 ppm, bevorzugt ≥ 1000 ppm, besonders bevorzugt ≥ 1500 ppm. Die Umsetzung von Adipinsäuredimethylester und 6-Hydroxycapronsäuremethylester mit 1,6-Hexandiol kann entweder rein thermisch erfolgen, oder durch Anwesenheit von katalytisch wirkenden Verbindungen, wie Säuren oder Basen. Bevorzugt ist die thermische Variante bei der die Temperatur ≥ 100°C ist und eine Kontaktzeit zwischen den Estern und 1,6-Hexandiol von wenigstens 5 Minuten bestehen muss. Bevorzugt sind Temperaturen ≥ 120°C sowie Kontaktzeiten von ≥ 10 Minuten.

In einer bevorzugten Ausführungsform, sofern Methylester in die Hydrierung eingesetzt wurden, ist vor dieser Kontaktzeit das Methanol bereits zumindest zu 50 % destillativ entfernt worden. Bevorzugt wird dies mit der Stufe zur Entfernung von Methanol kombiniert, die der eigentlichen Destillation nach Schritt 11 vorgelagert ist.

Das Stoffgemisch, das 1,6-Hexandiol, die Hochsieder, höhersiedenden Ester und gegebenenfalls Leichtsicher enthält, wird destillativ aufgetrennt. Bevorzugt werden dabei in einer ersten Destillationseinheit, z.B. einer kontinuierlich betriebenen Kolonne, Verbindungen die einen niedrigeren Siedepunkt als 1,6-Hexandiol wie z.B. die Leichtsieder wie Methanol destillativ abgetrennt. Nebenprodukte wie z.B. Wasser und Dimethylether fallen dabei zusammen mit dem Methanol an. Bei der Destillation wird die Energie bevorzugt über den Sumpf der Kolonne eingebracht, beispielsweise über einen Sumpfumlauf. Die Temperatur des Sumpfes sollte mindestens 100°C betragen. Es ist auch vorteilhaft, die Zulauftemperatur zur Kolonne oberhalb von 20 °C zu halten, beispielsweise auf dem Niveau bei dem die Hydrieraustäge anfallen, damit deren thermische Energie in der Kolonne mitgenutzt werden kann. Die mittlere Verweilzeit des 1,6-Hexandiols zusammen mit den vorgenannten Hochsiedern und höhersiedenden Estern beträgt in dieser Kolonne bei Temperaturen von mindestens 100°C zumindest 5 Minuten. Dieser 1,6-Hexandiol-haltige Strom wird vorteilhaft in einer weiteren Kolonne zu 1,6-Hexandiol mit einem Stickstoffgewichtsanteil von weniger als 5 ppm verarbeitet. Dabei kann z.B. eine Trennwandkolonne oder eine Kolonne mit einem Seitenabzug verwendet werden, bei der Leichtsieder wie z.B. 1-Hexanol, 1-Methoxy-6-hydroxyhexan zusammen mit möglichst wenig 1,6-Hexandiol über Kopf abdestilliert wird, über Sumpf Hochsieder und/oder höhersiedende Ester, die ebenfalls möglichst wenig 1,6-Hexandiol enthalten abzieht und über Seitenabzug, flüssig oder gasförmig 1,6-Hexandiol mit einem Stickstoffgehalt von weniger als 5 ppm abzieht. Bevorzugt enthält dieses 1,6-Hexandiol weniger als 3 ppm Stickstoff. Diese Kolonne wird bei Sumpftemperaturen über 100°C und mittleren Verweilzeiten von über 5 Minuten betrieben. Die stickstoffhaltigen Komponenten werden zumindest zu 50 % mit dem hochsiedenden Sumpfstrom ausgeschleust.

Anstatt der einen Kolonne mit Seitenabzug können auch zwei getrennte Kolonnen verwendet werden, wobei in der ersten Leichtsieder über Kopf entfernt werden, in der zweiten Kolonne dann das 1,6-Hexandiol von Hochsiedern und/oder höhersiedenden Estern abdestilliert werden. Zumindest in der ersten der beiden Kolonnen wird im Sumpf eine Temperatur von mindestens 100°C bei mittleren Verweilzeiten von mindestens 5 Minuten eingestellt

Zur Herstellung kleinerer Mengen 1,6-Hexandiols kann man auch sog. batch-Kolonnen verwenden, bei denen das Hexandiol diskontinuierlich aufgereinigt wird. Dabei werden zuerst Leichtsieder gegenüber Hexandiol abgetrennt, danach das 1,6-Hexandiol selbst. Im Sumpf verbleiben Hochsieder und höhersiedende Ester, die die Stickstoff-Komponenten enthalten.

Eine weitere Variante ist, nach der Hydrierung andere Carboxylgruppen haltige Komponenten ausser Carbonsäuren und Ester zuzusetzen. Diese Verbindung sind beispielsweise Aldehyde und Ketone, die mit dem 1,6-Hexandiol Verbindungen bilden, die höher sieden als das 1,6-Hexandiol selbst.

Wird Wasser als Lösungsmittel eingesetzt, ist es sinnvoll, die Adipinsäure selbst zu hydrieren. Als Hydrierkatalysator sind dann beispielsweise Co-, Re- und Ru-haltige Katalysatoren. Auch hier sollte der Umsatz an Carbonsäuren und/oder Estern nicht vollständig sein, so dass der Anteil an Carbonsäuren und/oder Estern im Hydrieraustrag bevorzugt über 500 ppm, besonders bevorzugt über 1000 ppm liegt.

Das so hergestellte 1,6-Hexandiol, dass einen Stickstoff (N)-Gehalt von weniger als 5 ppm aufweist, kann in jedes Herstellungsverfahren zur Herstellung von Polymeren, bei denen Diole verwendet werden, eingesetzt werden. Da das 1,6-Hexandiol nur einen N-Gehalt von weniger als 5 ppm aufweist, sind dadurch Polymere ohne Probleme erhältlich. Solches 1,6-Hexandiol kann für die Herstellung von Polyurethanen und Polyestern eingesetzt werden. Hierbei kann das 1,6-Hexandiol für die Polyurethane mit Diisocyanaten wie Hexamethylendiisocanat, Toluol-2,4-diisocyanat, Diphenylmethandiisocyanat, Isophorandiisocanat und 4,4'-Diisocyanatodicyclohexylmethan umgesetzt werden. Zur Herstellung von Polyestern kann solches 1,6-Hexandiol in Gegenwart von Dicarbonsäuren wie Bernsteinsäure, Maleinsäure, Fumarsäure, Glutarsäure, Adipinsäure, Dodecandisäure, Terephthalsäure, Ispphthalsäure und Phthalsäure eingesetzt werden.

Beispiele

Beispiel 1:

Adipinsäure, erhältlich als Produkt der Oxidation von Cyclohexanol/Cyclohexanon mit Salpetersäure, mit einem Gehalt von 4 ppm Stickstoff wird mittels eines sauren Ionentauschers als Katalysator (Amberlie IR 120) und Methanol zu Adipinsäuredimethylester verestert. Nach vollständiger Veresterung und Abtrennung von Ionentauscher sowie überschüssigem Methanol wird der Ester destilliert (18 mbar, Siedepunkt 115°C) und mit einer Reinheit von 99,98 % erhalten. Der Stickstoff (N)-Gehalt im Ester betrug 4 ppm. Der Adipinsäuredimethylester wird in der Gasphase bei 60 bar und 195 - 210°C an einem Kupfer-haltigen Katalysator hydriert. Dabei beträgt die Katalysatorbelastung 0,15 kg Esterfeed/Liter Katalysator pro Stunde. Der Reaktor ist ein Schachtreaktor, dem ein Verdampfer vorgeschaltet ist, bei dem der Feedstrom bei ca. 195 °C mit Hilfe eines Wasserstoffgasstroms verdampft wird. Der Wasserstoffgasstrom setzt sich aus Frischgas (4,5 Mol/Mol Adipinsäuredimethylester) und einem Kreisgasstrom (ca. 80 Mol Wasserstoff/Mol Feedstrom) zusammen. Nach dem Reaktor wird das gasförmige Gemisch abgekühlt und flüssige Produkte abgezogen. Der gasförmige Austrag wird mittels Kreisgaskompressors rückgeführt. Ein kleiner Teil des Gasstroms wird als Abgas ausgeschleust. Der Adipinsäuredimethylesterumsatz beträgt ca. 99,9 %. Durch den Abgasstrom ging etwas Methanol verloren. Die gesammelten Austräge (ca. 30 Gew.-% Methanol, ca. 68 Gew.-% 1,6-Hexandiol, ca. 0,5 Gew.-% 6-Hydroxycapronsäuremethylester und 0,06 Gew.-% 6-Hydroxycapronsäurehexandiolester, ca. 0,3 Gew.-% Hexanol, 0,1 Gew.-% Adipinsäuredimethylester, Rest jeweils unter 0,1 Gew.-%) weisen einen N-Gehalt von 5 ppm auf und werden destillativ aufgearbeitet. Dabei wird zuerst überwiegend Methanol bei Sumpftemperaturen bis 140 °C und Drücken von 1013 mbar absolut bis 100 mbar innerhalb einer Stunde entfernt. Der verbleibende Sumpf (ca. 0,08 Gew.-% Adipinsäure-1,6-hexandiol-methylester, 0,02 Gew.-% Adipinsäuredi-1,6-hexandiolester, 0,3 Gew.-% 6-Hydroxycapronsäure-1,6-hexandiolester) wird innerhalb von zwei Stunden diskontinuierlich in einer Destillationskolonne (1 m Füllkörperkolonne, Rücklaufverhältnis 5, kein Lufteintritt) bei 100 mbar absolut und Sumpftemperaturen von ca. 185 °C fraktioniert destilliert. Nach Abtrennung von Leichtsiedern wie Restmethanol und Hexanol gewinnt man 1,6-Hexandiol in einer Destillationsausbeute von ca. 90 % mit einer Reinheit von 99,9 % und einem N-Gehalt von 1 ppm. Der N-Gehalt im verbleibenden Sumpf liegt bei 15 ppm.

Vergleichsbeispiel 1:

Beispiel 1 wird wiederholt, mit dem Unterschied, dass in der Hydrierung zusätzlich ein zweiter Reaktor eingebaut wird, der in Ausmaß und Inhalt dem ersten entspricht und nach dem ersten Reaktor durchströmt wird. Entsprechend geht die Katalysatorbelastung auf 0,75 zurück. Der Umsatz an Adipinsäuredimethylester war praktisch vollständig, neben Methanol und Hexandiol fanden sich 6-Hydroxycapronsäureester in Form von Methyl- und Hexandiolester in Mengen unter 0,03 Gew.-% , ca. 0,6 Gew.-% Hexanol, Rest jeweils unter 0,05 Gew.-%. Der Austrag weist wiederum einen N-Gehalt von 5 ppm auf. Es wird wie in Beispiel 1 1,6-Hexandiol weiter aufbereitet. Das resultierende 1,6-Hexandiol wies einen N-Gehalt von 5 ppm, das Sumpfprodukt von 7 ppm auf.

## Patentansprüche

1. Verfahren zur Herstellung von 1,6-Hexandiol umfassend folgende Schritte

I) Bereitstellung einer Mischung, die 1,6-Hexandiol enthält

II) Destillation dieser Mischung aus Schritt I
III) Auffangen eines 1,6-Hexandiols mit einem Stickstoffanteil von weniger als 5 ppm,
IV) Ausschleusen von zumindest 50 % der stickstoffhaltigen Komponenten mit dem hochsiedenden Sumpfstrom,

wobei vor und/oder während der Destillation in Schritt II mehr als 500 ppm an Carbonsäuren und/oder Ester enthalten sind, die einen höheren Siedepunkt als 1,6-Hexandiol aufweisen und bei Temperaturen von ≥ 100°C wenigstens 5 min lang in Kontakt mit dem 1,6-Hexandiol stehen.

2. Verfahren nach Anspruch 1, wobei die Carbonsäuren und/oder Ester, die einen höheren Siedpunkt als 1,6-Hexandiol aufweisen, vor der Destillation nach Schritt II zur Mischung gegeben werden.

3. Verfahren nach einem der Ansprüche 1 bis 2, wobei die Destillation in Abwesenheit von Sauerstoff durchgeführt wird.

4. Verfahren nach einem der Ansprüche 1 bis 3, wobei die Destillation im Bereich von 10 bis 3000 mbar durchgeführt wird.

5. Verfahren nach einem der Ansprüche 1 bis 4, wobei die Destillation in Schritt II) batchweise durchgeführt wird.

6. Verfahren nach einem der Ansprüche 1 bis 4, wobei die Destillation in Schritt II) kontinuierlich durchgeführt wird.

7. Verfahren nach einem der Ansprüche 1 bis 6, wobei die Umsetzung in Schritt II) in Gegenwart von Estern durchgeführt wird.

## Claims

1. A process for preparing 1,6-hexanediol, which comprises the following steps

I) provision of a mixture comprising 1,6-hexanediol
II) distillation of this mixture from step I
III) collection of a 1,6-hexanediol having a nitrogen content of less than 5 ppm,
IV) discharge of at least 50% of the nitrogen-comprising components with the high-boiling bottom stream,

wherein more than 500 ppm of carboxylic acids and/or esters which have a boiling point higher than that of 1,6-hexanediol and are in contact with the 1,6-hexanediol at temperatures of z 100°C for at least 5 minutes are comprised before and/or during the distillation in step II.

2. The process according to claim 1, wherein the carboxylic acids and/or esters which have a boiling point higher than that of 1,6-hexanediol are added to the mixture before the distillation in step II.

3. The process according to either claim 1 or 2, wherein the distillation is carried out in the absence of oxygen.

4. The process according to any of claims 1 to 3, wherein the distillation is carried out in the range from 10 to 3000 mbar.

5. The process according to any of claims 1 to 4, wherein the distillation in step II) is carried out batchwise.

6. The process according to any of claims 1 to 4, wherein the distillation in step II) is carried out continuously.

7. The process according to any of claims 1 to 6, wherein the reaction in step II) is carried out in the presence of esters.

**Revendications**

1. Procédé de préparation de 1,6-hexanediol comprenant les étapes suivantes :

   I) la préparation d'un mélange contenant du 1,6-hexanediol,
   II) la distillation de ce mélange de l'étape I),
   III) le recueillement d'un 1,6-hexanediol ayant une proportion d'azote inférieure à 5 ppm,
   IV) l'évacuation d'au moins 50 % des composants azotés avec le courant de fond de point d'ébullition élevé,

   dans lequel, avant et/ou pendant la distillation à l'étape II), plus de 500 ppm d'acides carboxyliques et/ou d'esters sont contenus, qui présentent un point d'ébullition supérieur au 1,6-hexanediol et qui sont en contact avec le 1,6-hexanediol pendant au moins 5 minutes à des températures $\geq$ 100 °C.

2. Procédé selon la revendication 1, dans lequel les acides carboxyliques et/ou les esters qui présentent un point d'ébullition supérieur au 1,6-hexanediol sont ajoutés au mélange avant la distillation selon l'étape II).

3. Procédé selon l'une quelconque des revendications 1 à 2, dans lequel la distillation est réalisée en l'absence d'oxygène.

4. Procédé selon l'une quelconque des revendications 1 à 3, dans lequel la distillation est réalisée dans la plage allant de 10 à 3 000 mbar.

5. Procédé selon l'une quelconque des revendications 1 à 4, dans lequel la distillation à l'étape II) est réalisée de manière discontinue.

6. Procédé selon l'une quelconque des revendications 1 à 4, dans lequel la distillation à l'étape II) est réalisée de manière continue.

7. Procédé selon l'une quelconque des revendications 1 à 6, dans lequel la réaction à l'étape II) est réalisée en présence d'esters.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9731882 A1
- US 6426438 B
- DE 10112117 A1
- DE 101112114 A1